# EUROPEAN PATENT SPECIFICATION

(11) **EP 3 226 957 B1**
(45) Date of publication and mention of the grant of the patent: **02.02.2022**
(21) Application number: 15865004.4
(22) Date of filing: 03.12.2015
(51) Int. Cl.: A61M 35/00

(54) **DISPENSER**
SPENDER
DISTRIBUTEUR

(30) Priority: 03.12.2014 US 201462087015 P
(43) Date of publication of application: 11.10.2017
(73) Proprietor: 623 Medical, LLC, Morrisville, NC 27560 (US)
(72) Inventor: LOZEVSKI, Jonathan, Morrisville, NC 27560 (US)
(74) Representative: Studio Torta S.p.A.
(86) International application number: PCT/US2015/063759
(87) International publication number: WO 2016/090152

(56) References cited:
- WO-A1-2005/107834
- WO-A1-2007/028992
- WO-A1-2008/093372
- GB-A- 1 255 666
- US-A- 3 429 310
- US-A- 5 516 006
- US-A1- 2002 117 518
- US-A1- 2007 044 488
- US-A1- 2008 142 036
- US-A1- 2013 103 127
- US-A1- 2013 184 677
- US-A1- 2013 218 093
- US-A1- 2014 188 094
- US-B1- 7 341 056
- US-B2- 8 535 276

## Description

### Technical Field

The present disclosure relates to a dispenser.

### BACKGROUND

Injections are employed millions of times daily all over the world to deliver medicines into people as well as animals. Many times, injections are made in areas of the body that are significantly sensitive to pain that may be quite painful as the needle is inserted. Cooling of the skin in the vicinity of injection site using vapocoolant is known, but existing dispensing techniques and devices can be cumbersome to employ, may cause injury to the injection site, and employ flammable or environmentally unfriendly materials. Deploying a sufficient amount and duration of vapocoolant while maintaining a desired distance from the injection site is but one of the difficulties inhibiting wide-spread acceptance of such devices and methods. Manipulating and injecting within a short window of time after deploying the vapocoolant is another drawback of existing systems. Coupling of dispensing devices to injection devices such as syringes is known, however, customization of such devices leads to increased costs and end-user dissatisfaction. Other applications of dispensing inks, medicament, indicators, and the like, have similar room for improvement in the design, ease-of-use, and simplicity of devices useful for this purpose. The present disclosure provides an improved dispenser device.

WO 2007/028992 discloses an inhaler provided with a closure which may be moved between a closed and open position in order to cover and uncover a mouthpiece.

US 3429310 discloses an aerosol inhalating device and US 5516006 discloses a nasal dispenser for dispensing medications.

WO 2005/107834 discloses an apparatus for mitigating discomfort and/or pain upon injection, which allows to locally cool the skin.

US2007044488 is about a skin cooling device provided with a compressible housing, made of two portions and enclosing a container, and a removable cover.

### SUMMARY

A vapocoolant dispenser, comprising:
a housing (200) having a first portion (201) and a second portion (202) coupled with the first portion defining a collapsible chamber (99);
a container (220) configured to contain contents under pressure, the container positioned in the housing (200);
a valve member (232) for selectively opening the container, the valve member comprising: an actuator portion (234) configured to extend from the container; and
a seal member (236) sealably engaged with the container; and
a stop member (210) positioned in the second portion (202) of the housing (200) and configured to engage the actuator portion (234);
such that when the collapsible chamber is collapsed, the actuator portion is engaged by the stop member, disengaging the seal member from the container for releasing the contents,
wherein one of the first portion and the second portion has an inner perimeter of larger dimension than an outer perimeter of the other one of the first portion and the second portion, and wherein the first portion is configured to translate along a common longitudinal axis with that of the second portion so as to collapse the collapsible chamber, wherein the container is fixedly positioned in the second portion of the housing; a cover assembly (218) is releasably attached to the second portion (202)
wherein the cover assembly comprises a prep pad (250) having antiseptic;
wherein the cover assembly comprises rim (221) configured to engage with tab (214) of first portion (201) and the internal surface of the cover assembly (218) and the external surface of the second portion (202) are formed with mating retaining members (215); and
a rigid skirt (207) integral within and extending about an annular edge of the second portion; wherein the cover assembly is released with the following mechanism: when the housing portion (201) is depressed to begin the collapse of chamber (99), the tab members (214) engage the rim (221) and cause the retaining members (215) to release such that the cover assembly (218) is released from the second housing portion (202).

Preferably, the dispenser further comprises an absorbent material having an inward facing side and an outward skin-facing side, the absorbent material positioned distally from the valve member to receive the contents on the inward facing side.

Preferably, the stop member comprises a surface having a recess (216) dimensioned to receive at least a portion of an outlet (230) and at least a portion of the valve member (232).

The rigid skirt preferably terminates in an annular ring (209), and where the dispenser further comprising an absorbent material fixedly held to the annular ring, the absorbent material having an inward facing side and an outward skin-facing side, the absorbent material positioned distally from the valve member and configured to receive the contents on the inward facing side.

Preferably, the cover assembly is configured to remain connected to the housing upon release.

Preferably, the dispenser is configured to transition to:
a first state wherein the contents are contained within the container and the cover assembly is released from the second portion (202) of the housing (200); and
a second state wherein the first and the second portions are urged in proximity with each other so as to collapse the collapsible chamber (99) and engage the actuator ( 234) with the stop member (210) disengaging the seal member (236) from an outlet (230) and allowing an amount of the contents to be released.

The cover assembly is preferably configured to disengage from the housing prior to release of the contents.

The contents preferably comprise a vapocoolant and a metered amount of the vapocoolant is released or wherein an amount of vapocoolant is continuously released.

### BRIEF DESCRIPTION OF THE DRAWINGS

FIG. 1 illustrates a dispenser according to an embodiment of the disclosure, which is not part of the claimed invention.
FIGs. 2 and 3 are a section view taken along line 2-2 of FIG. 1 and an exploded view thereof, respectively.
FIG. 4 illustrates a dispenser according to the present invention.
FIGs. 5 and 6 are a section view taken along line 5-5 of FIG. 4 and an exploded view thereof, respectively.
FIGs. 7 and 7A represents an exploded view of the dispenser of FIG. 4 and an alternate embodiment thereof, showing operation of the dispenser.
FIGs. 8 and 8A illustrates dispensers according to yet another embodiment of the disclosure, which is not part of the present invention.
FIGs. 9 and 10 are a section view taken along line 9-9 of FIG. 8 and an exploded section view thereof, respectively.
FIG. 11 illustrates a dispenser according to another embodiment of the disclosure, which is not part of the present invention.
FIG. 12 is a top view of the dispenser of FIG. 11.
FIG. 13 is a section view taken along line 13 - 13 of Fig. 12.
Fig. 14 is an exploded view of the dispenser of FIG. 11.
FIG. 15 illustrates a dispenser according to yet another embodiment of the disclosure, which is not part of the present invention.
FIG. 16 is a top view of the dispenser of FIG. 15.
FIG. 17 is a section view taken along line 17 - 17 of Fig. 16.
Fig. 18 is an exploded view of the dispenser of FIG. 15.
Fig. 19 is a view of one embodiment of the disclosure, which is not part of the present invention, illustrating a use of the device.

### DETAILED DESCRIPTION

The dispenser according to the present invention comprises a housing arranged to provide a collapsible chamber sized to receive a container comprising a dispensable composition. In one aspect, the dispensable composition provides a headspace pressure in the container greater than atmospheric pressure at ambient temperature, such that the dispensable composition is under a pressure without the addition or assistance of a pressurized gas. In one aspect, the dispenser comprises a container comprising one or more vapocoolants.

The housing is formed by two portions the first portion arranged to receive a portion of the second portion so that when they are assembled together the two portions create the collapsible chamber configured for receiving the container. The housing is arranged to receive a container and to allow movement of the container such that the container's valve assembly is actuated for releasing the contents thereof.

In one aspect, the container comprises a valve member. The valve member may include an elongated tube having an axis of elongation along which an outlet is formed. When the actuator is actuated by the translation of the two housing portions relative to each other, the valve is urged into the container and access to the outlet is provided, allowing release of the vapocoolant within the container. The valve member may include a tilt valve.

The dispensing device is configured with a housing comprising a collapsible chamber sized to receive a container such that when the housing portions are traversed in opposite directions along a common longitudinal axis collapsing the collapsible chamber, causing the container to move in a direction towards a stop member that engages actuator portion of the valve member.

In one aspect, the present devices are intended for providing topical application of a vapocoolant to skin. By skin, it is intended to include intact mucous membranes, the oral cavity, nasal passage ways and the lips. In one aspect, the present device is intended to provide pain management associated with injections, e.g., pre-injection anesthesia, including but not limited to venipuncture, IV starts, minor surgical procedures and the temporary relief of pain from injuries, e.g., topical anesthesia, such as sprains, bruising, cuts, abrasions, and insect bites. The present device can also provide myofacial pain management. The dispenser can be configured to dispense other compositions, such as medicaments, dissolved or dispensed or distributed in low boiling solvents, such as ether or fluorocarbons, for non-invasive peroral (through the mouth), topical (skin), transmucosal (nasal, buccal/sublingual, vaginal, ocular and rectal) and inhalation dispensing to infants or adults. Other compositions that can be dispensed by the present dispenser can include removable or indelible inks, e.g., for tattooing or otherwise marking skin with indicia, e.g., phosphorescent inks.

While it is understood that the dispensable composition can be other compositions, exemplary examples and discussions that now follow recite the use of a vapocoolant to describe manner and method of the presently disclosed dispenser.

When the dispenser is employed with a vapocoolant, e.g., as a "vapocoolant dispenser," it is released and caused to contact the skin, producing an instantaneous cooling effect upon contact. In one aspect, the dispenser is configured to provide the vapocoolant in the form of an aerosol, in either a mist or stream spray. Upon contact with the skin or mucosal membranes, the dispensed vapocoolant evaporates immediately due to the low evaporation rate and the range of temperature associated with skin, causing an instantaneous cooling effect at the application site by the immediate evaporation of the vapocoolant. The rapid reduction of temperature of the application site created by the dispensed vapocoolant is believed to decrease nerve conduction velocity of the C fibers and A-delta fibers that make up the peripheral nervous system, thus interrupting the nociceptive inputs to the spinal cord, among other things. The amount and/or duration of the cooling produced is related to the vapocoolant used, ambient temperature and altitude, skin temperature, the shape of stream and/or the distance of the dispensing vapocoolant from the application site, the duration of the dispensing of the vapocoolant, among other variables. For example, as the distance from the application site is increased, the dispersion of the vapocoolant (either as droplets, mist, and/or stream) is increased along with the total surface area about the application site. This results in a decrease of the size of the vapocoolant droplets, thus increasing their evaporation rate and improving the effective temperature change of the skin. This distance-cooling rate relationship has physical limits of course, and the present dispenser can be configured to optimize the distance the released vapocoolant travels before contacting the application site, as further discussed below. In one aspect, the duration the vapocoolant is caused to contact the skin is controlled, as well as the combination of distance and duration, as further discussed below. In one aspect, the amount and duration of exposure to skin to the vapocoolant is such that the skin temperature does not go below 40°F (4.4°C). In one aspect the amount and duration of exposure to skin to the vapocoolant is controlled by configuring the device to dispense the vapocoolant from a predetermined distance relative to the surface of the skin as described further below. The predetermined distance can be determined based on the nature of the vapocoolant and the expected duration of contact of the skin with the vapocoolant.

The container can be comprised of any material suitable for containing vapocoolant, which typically involves pressure slightly above that of atmospheric. Suitable materials include metals such as stainless steel and aluminum, plastics, reinforced plastics, glass, and ceramics.

In one portion of the housing a suitable structure is provided that receives the actuator portion and renders the valve substantially stationary after traversal of a distance by one or both of the two housing portions. Further traversal of the container together with the housing portions after the actuator portion of the valve member is rendered stationary causes the valve portion of the valve member to open so as to affect dispensing of the vapocoolant from within the container. In other aspects, a restoring force of a spring incorporated in the valve, or in the housing, restores the container and valve to its previous un-actuated position. If desired, a cam device may be associated with the housing so that a prescribed metered dose of vapocoolant is dispensed each time the two housing portions are collapsed, regardless of how long it is held in the depressed position.

The dispenser of the present disclosure is intended, in one variation, to be used continuously until the container is depleted of vapocoolant and then discarded. The device may be used multiple times and, even, multiple patients. Of course, the device may be used solely with a single patient and discarded after such use.

The two housing portions of the housing can be assembled about the container. In one aspect the housing portions are configured to be engaged for rotation relative to one another about a common longitudinal axis so as to collapse the collapsible chamber. In another aspect the two portions are configured to be translated in opposite directions relative to a common longitudinal axis.

One of the housing portions may incorporate a pair of opposed wing-like appendages sized and configured to be engaged by fingers of the user, with the user's thumb being employed to depress one of the housing portions.

The housing can be made of metal, glass, or ceramic. In one aspect, the housing and any or all of its components can be made of plastic, e.g., by any desired method including injection molding, compression molding, rotational molding, 3-D printing, and the like. The container can be of any material suitable for a vapocoolant, e.g., a plastic, metal, glass, or ceramic material capable of containing a liquid and/or gas at a pressure above atmospheric. The container can be sized to hold 0.1-1000 mL of vapocoolant. In one aspect, the container is sized to contain about 3.5 fl. oz. (103.5mL). In other aspects, the container is sized to hold 0.5 mL, 1 mL, 2 mL, 5 mL, 10 mL, 25 mL, 50 mL, 75 mL, 200 mL, 500 mL, or 750 mL or more of vapocoolant.

The vapocoolant can be any liquid or combination of liquids having properties suitable for use as a vapocoolant. Such properties would include, low toxicity, low flammability and low combustibility, and/or includes materials having suitable boiling point and vapor pressure at typical or envisioned end-use temperature/pressure conditions. In one aspect, one or more halogenated hydrocarbons can be used as vapocoolants. In other aspects, suitable vapocoolants include one or more refrigerants as defined by American Society of Heating, Refrigerating and Air Conditioning Engineers (ASHRAE) as hydrochlorofluoroolefin, hydrochloric carbon hydrochloroolefin, hydrocarbon, hydroolefin, perfluorocarbon, perfluroolefin, perchlorocarbon, perchloroolefin, or mixtures thereof, for example, 1,1,1,3,3-pentafluoropropane, and 1,1,1,2-tetrafluoroethane mixture. One or more chemical compounds, e.g., medicaments, can be at least partially dispersed or dispensed or dissolved in the vapocoolant. In one aspect, the contents of the container, without pressurized air or inert gas, provides its own head-space pressure of above ambient atmospheric pressure, e.g., 1.01 to about 3 atmospheres at ambient temperature. For example, 1-3 atmospheres of pressure (about 15 psi to about 45 psi) is achievable by one or more vapocoolants or combinations of one or more vapocoolants and one or more hydrofluorocarbon alkanes (HFA), which can be safely contained in the container of the present disclosure. This head-space pressure without pressurized air or other gas is obtainable, for example, by providing a 95% mixture of a vapocoolant and a 5% mixture of a HFA, e.g., 95 wt.% of 1,1,1,3,3-Pentafluoropropane and 5 wt.% of 1,1,1,2-Tetrafluoroethane. Other combinations are possible to achieve the head-space pressure and encompassed by the present disclosure. Vapocoolants can include, without limitation, one or more non-halogen containing low boiling fluids suitable for topical skin application, provided that the non-halogen containing the fluid is capable of operating as a self-propellant by providing a suitable pressure for discharge in a vapor space above the liquid supply of the vapocoolant.

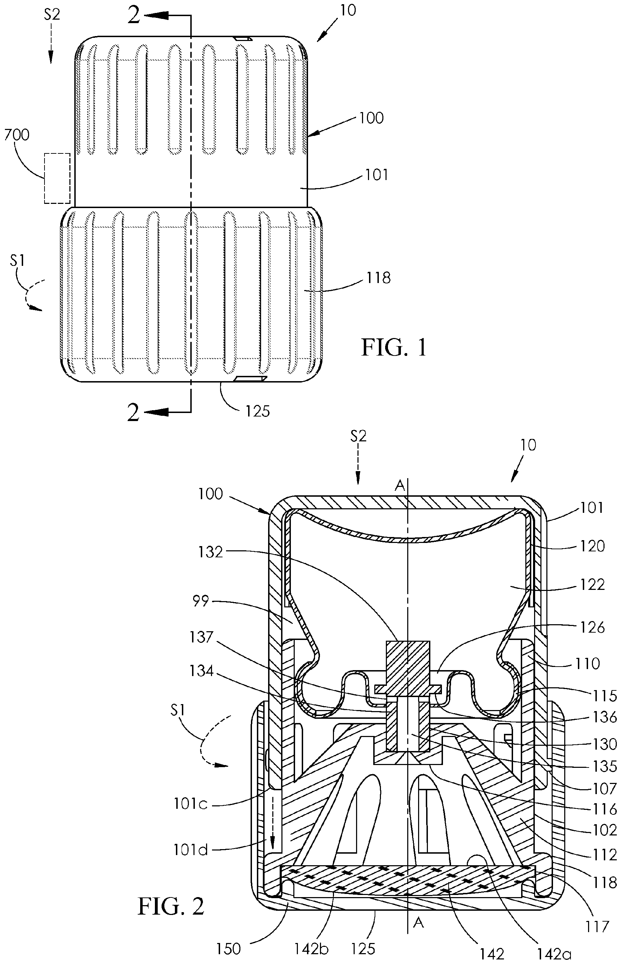

With reference, now, to FIGs. 1 - 3, an embodiment of a dispenser is generally designated by the reference numeral 10, depicted as having an elongated housing 100 including a first and a second housing portions, 101 and 102, respectively, configured for coupling together, where, as exemplarily shown, each portion is shown having essentially a cylinder shape, where portion 102 is of an exterior diameter that is less than the inner diameter of portion 101 such that portion 102 is slidably received in portion 101. The housing portions may have other shapes than the cylindrical shapes shown in the drawings. In this arrangement, the two housing portions 101, 102 are couplable together to form an inner collapsible chamber 99 that is collapsible upon translation of first housing portion 101 towards second housing portion 102 as indicated by arrow S2. The dispenser occupies at least a first state in which the cover assembly is removed and chamber 99 is not collapsed and a second state in which the housing portions are moved toward one another and the chamber is collapsed to dispense the contents of the container.

Integral with second housing portion 102 is stop member 112 providing recess 116, essentially centered along the longitudinal axis A-A of the housing 100. The second housing portion 102 has a projecting wall 110 that essentially surrounds stop member 112 and recess 116. Projecting wall 110 is closely received by the interior surface of first housing portion 101 upon assembly of housing 100. An outwardly extending lip (not shown) on second housing portion 102 engages an abutment surface (not shown) formed in a groove on first housing portion 101 to prevent vertical separation of the portions from each other but to allow translation of the portions toward each other. Lip and groove can include sealing means, such as an o-ring. Distal end 101c of first housing portion 101 traverses along outer diameter of second housing 102 during the dispensing operation, as indicated by arrow 101d. Chamber 99 receives container 120 that may have contents 122, e.g., vapocoolant, contained therein. The contents of the container 120 are under pressure such that the pressure inside of the container 120 is greater than the ambient pressure.

When present in the collapsible chamber 99 of the dispenser 10, container 120 includes valve member 132 positioned in opening 130 of container 120. Valve member 132 comprises actuator 134 for containing and allowing controlled release of contents 122 from container 120 (valve member 132 is shown in a closed position in FIG. 2). The valve member 132 comprises seal member 136 that cooperates with actuator 134 to release contents 122 through orifice or nozzle 135 formed in stop member 112. One or more o-rings (not shown) can be positioned in the opening 130 in sealing arrangement with the seal member 136. One or more springs (not shown) can be used to assist in restoring the valve member 132 to the first closed position. In one aspect, conventional press-down valves with continuous actuation that function in both the upright or upside down position may be used, e.g., valves sold by Coster Technologie Speciali S.p.A., Italy.

When present in the collapsible chamber 99 of the dispenser 10, container 120 is disposed such that actuator 134 is received by the recess 116. Container 120 typically has sloping side walls terminating in a crimp 115 providing an outlet structure 126 associated with and securing the valve member 132 in the container 120. Valve member 132 can have an opening 137 in fluid communication with the contents of the container 120 when the valve member 132 is moved and seal member 136 is moved away from opening 130. Container 120 is at least partially surrounded by projecting wall 110 such that the container can traverse essentially in a straight path along the longitudinal axis A-A of the housing 100 when the dispenser is moved from the first state to the second state and the collapsible chamber 99 is collapsed.

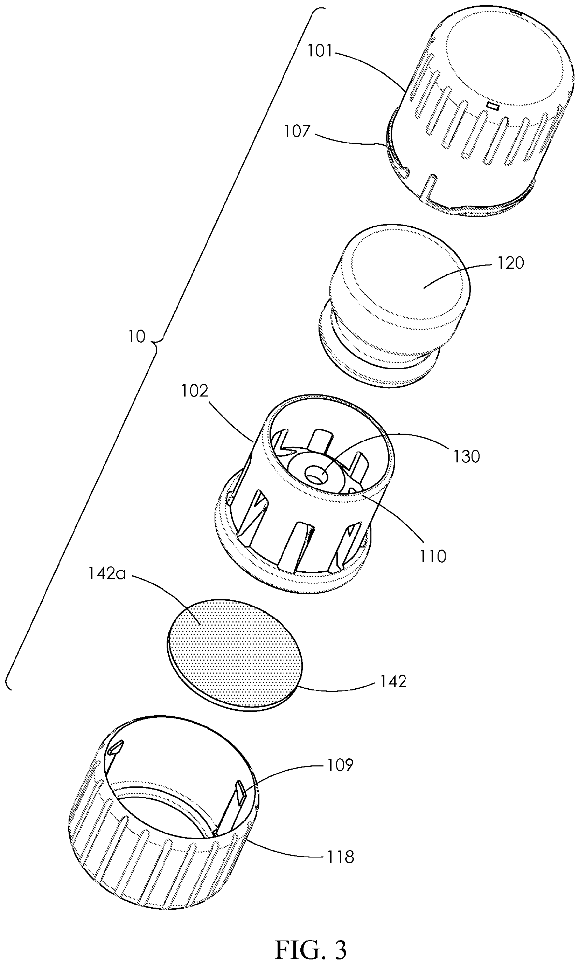

As depicted in FIGs. 2 and 3, first housing portion 101 comprises engagement member 107 that cooperates with corresponding engagement members 109 of cover assembly 118. Engagement member 107 can be arranged in a helical-like or screw-like track configuration to provide screw-like rotation (as depicted by arrow S1) of the corresponding two portions so as to affect release of cover assembly 118. Further, the engagement members may be reversed with the engagement member 109 on housing portion 101 and engagement member 109 on cover assembly 118. Engagement members 107 and 109 can be of tongue and groove configuration, cam and lobe configuration or conventional nut and bolt-like threads about the corresponding outer and inner diameters of the respective housing portion 101 and cover assembly 118. The engagement members are arranged such that rotation of the housing portion 101 relative to cover 118 releases cover 118 from the dispenser.

Optionally, second housing portion 102 can comprise soak pad 142 of absorbent material, coupled to the distal end of housing portion 102. Soak pad 142 has an upper surface 142a and lower surface 142b where the upper surface is configured to be substantially perpendicular to orifice (or nozzle) 135 so as to receive expelled contents 122. Soak pad 142 is shown secured to housing portion 102 via groove 117 sized to accept the perimeter of soak pad 142. Cover assembly 118 can comprise peel-away tab, for preventing contamination and/or maintain sterility of the interior of dispenser 10 prior to use. Cover assembly 118 may optionally include a prep pad (not shown), e.g., soaked in alcohol or antiseptic that can be used prior to the release of cover assembly 118 to treat a site of the subject prior to dispensing the contents of container 120.

Thus, in one aspect, dispenser 100 provides for the "prep" of a site on a subject, using prep pad that is exposed, e.g., by pulling off a peel-away tab (not shown) attached to cover 118. Dispenser 10 with prep pad exposed can be used to sterilize a site of a subject. The prep pad can also be configured to function as a bandage to be used after injection for example.

After use of the prep pad, or if no prep of the site is required, dispenser 10 is configured such that rotation of cover assembly 118 relative to first housing portion 101 provides for release of cover assembly 118, and optional prep pad from dispenser 10. This rotation can also facilitate a first state of activation. In one aspect of the present disclosure, the combination of cover and optional prep pad and housing with container, actuator and optional absorbent pad constitute an assembly.

In a second state, vertical translation of the first housing portion 101 along longitudinal axis A towards second housing portion 102 (in the direction of arrow 101a), e.g., using a thumb or other digit of a hand, causes valve member 132 to open as the position of the valve member is fixed against the stop member and the container is moved relative to the valve member to allow release of contents of container 120. Thus, dispenser 100 may be configured for a two-stage activation process (rotation-translation) where after prep pad 150 is exposed and used at the application site, rotation of first housing portion 101 relative to cover assembly 118 provides for release of the cover assembly 118 (and coupled prep pad 150), and subsequent translation of the first and second housing portions 101, 102 towards each other along axis A, causes contents of container 120 to be released towards application site of subject.

In another aspect, optional temperature sensor 700, is shown secured to outer surface of first housing portion 101 and arranged to detect, for example, infrared heat from the subject's skin in proximity to the site of application of the contents of the container, and to display a temperature or indicate a threshold temperature, e.g., red/green light or green light during use of the dispenser. Sensor 700 can be a user-operated, passive sensor with a threshold temperature set point. Sensor 700 can be a quantum detector, thermopile sensor, a microelectromechanical systems (MEMS) ultra-low power sensor, or thermocouple detector. For example, a TMP006 IR-thermopile sensor (Texas Instruments) with battery power source and on/off switch can be coupled to a green and/or red light emitting diode (LED) and mounted in a housing affixed to the first housing portion 101.

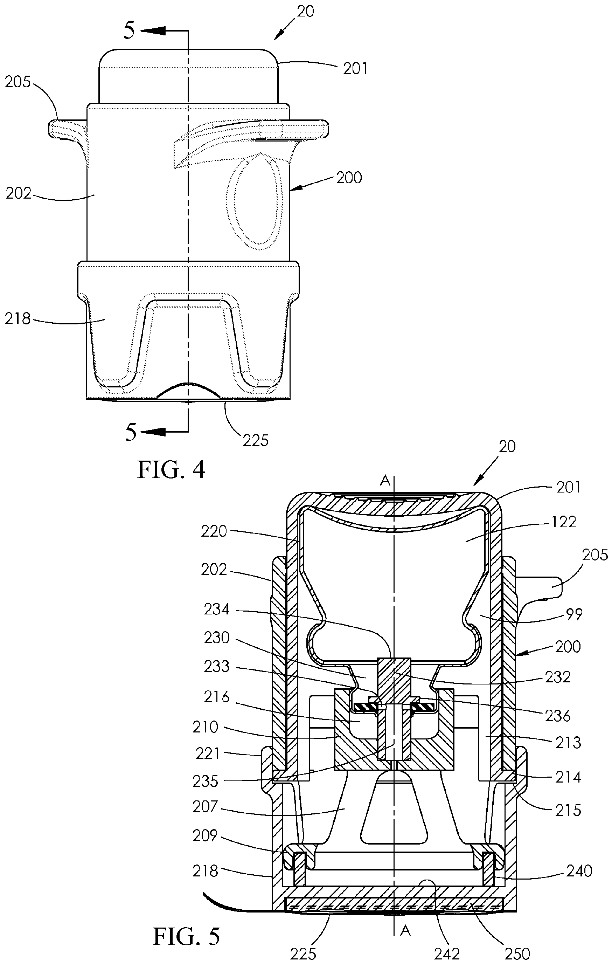

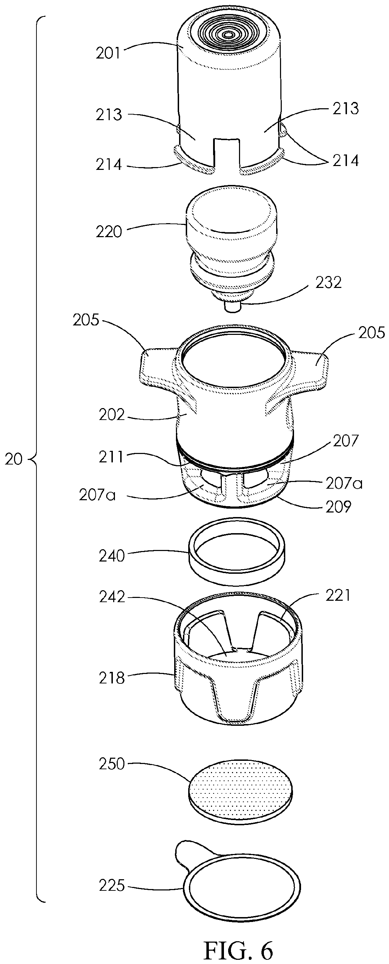

With reference, now, to FIGS. 4 - 6, the dispenser object of the present invention is generally designated by the reference numeral 20, depicted as having an elongated housing including a first and a second housing portions, 201 and 202, respectively, configured for coupling together, where, as exemplarily shown, each portion is essentially of a cylinder shape, where one portion is of an exterior diameter that is less than the inner diameter of the other portion. The portions may have shapes other than cylindrical. Contained in one housing portion is stop member 210 having recess 216 spatially positioned along the longitudinal axis A-A of the housing 200, the stop member 210 having a proximally extending rigid skirt 207 that distally terminates in annular ring 209. Rigid skirt 207 supports soak pad 250 suspended about ring 240 that is secured to the distal end of rigid skirt 207 at annular ring 209.

In this arrangement of dispenser 20, the two portions are couplable together and with the stop member 210, provide an inner collapsible chamber 99 that can transition from a first state to a second state where the collapsible chamber 99 collapses upon translation of the two portions toward each other as discussed further below. The housing portion 201 provides for receipt of container 220 that may have contents 122 contained therein. When present in the collapsible chamber 99 of the dispenser 20, container 220 includes valve member 232 comprising actuator 234 for containing and allowing controlled release of contents 122 from container 220 (valve member 232 is shown in a partially open position). The valve member 232 comprises seal member 236 that cooperates with actuator 234 to release contents 122 through orifice 235 formed in stop member 210. Valve member 232 can have an opening 233 in fluid communication with the contents of the container 220 when seal member 236 is moved away from outlet 230. One or more o-rings (not shown) can be positioned around the opening 230 of container 220 in sealing arrangement with the seal member 236. One or more springs (not shown) may be used to assist in restoring the valve member 232 to the closed position. When present in the collapsible chamber of the dispenser, container 220 is disposed such that actuator 234 is received by recess 216. Container 220 typically has sloping side walls and is positioned in housing 200 such that the container 220 can traverse essentially in a straight path along the longitudinal axis A-A of the housing 200 when the collapsible chamber 99 is collapsed.

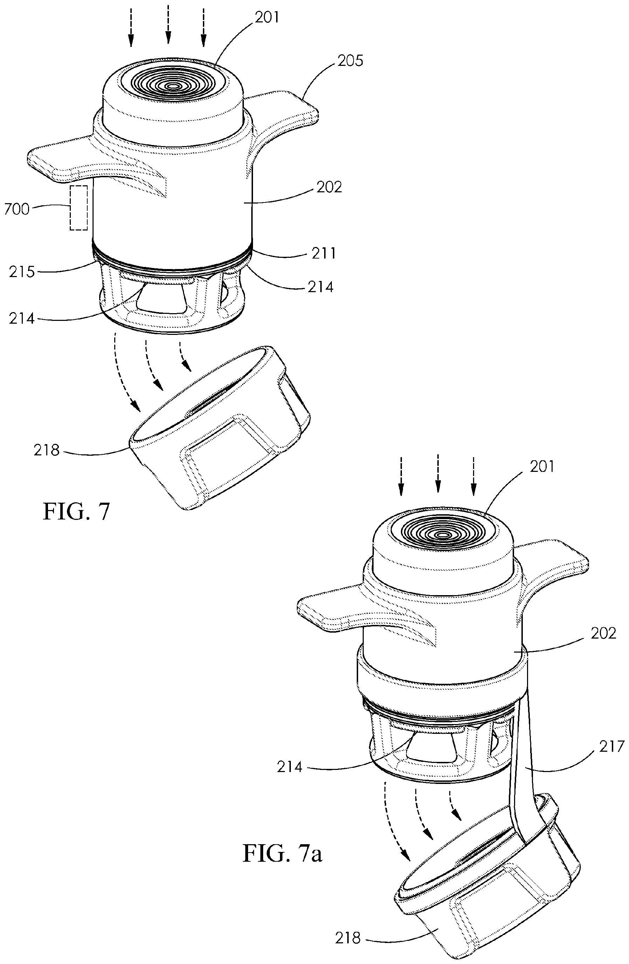

First housing portion 201 includes projections 213 with tab members 214 that engage cover member 218 through the openings 207a in extending rigid skirt 207 about an annular edge 211 of second housing portion 202. Tab members 214 are configured to engage with a proximal rim 221 of cover member 218 when the dispenser is moved from the first state to the second state such that in the first state, the cover member 218 is securably held on the second housing portion 202. The internal surface of the cover member 218 and the external surface of the housing portion 202 may be formed with mating retaining members 215 such as mating protrusions and recesses such that the cover member 218 is releasably held on the housing portion 202. The configuration of tab members 214, stop member 210, and cover member 218 provides for the release of the cover member 218 during transition to the second state prior to release of vapocoolant. When the housing portion 201 is depressed to begin the collapse of chamber 99, the tab members 214 engage rim 221 and cause retaining members 215 to release such that the cover member 218 is released from the second housing portion 202 as illustrated in FIG. 7. In this configuration and arrangement, the dispenser 20 is provided with the valve member 232 and nozzle covered by the cover member 218 and provides for aseptic or semi-aseptic assembly. The distal end of cover member 218 optionally may include a prep pad 250, e.g., soaked in alcohol or antiseptic.

Prep pad 250 and/or ring 240 can be employed to contain and release at least a portion thereof to the skin a temperature sensitive thermochromic material capable of changing color with change of temperature. The ink-like (or paint-like) material can be "stamped" on the skin application site and the dispensing of vapocoolant onto the skin application site would be visibly indicated to the user as a change in color. Such thermochromic materials are known, such as the leuco dyes (spirolactones, fluorans, spiropyrans, and fulgides for example), and liquid crystals (cholesteryl nonanoate or cyanobiphenyls, for example).

An optional pull tab 225 can hermetically or otherwise seal the distal end of cover member 218, preventing contamination and covering the prep pad 250, if desired. Prior to use, the end user can remove the pull tab 225, mark the skin with thermochromic material from the prep pad 250, begin to transition from the first state to the second state so as to release the cover member 218 as depicted in FIG. 7, to allow dispensing of contents when the dispenser is urged to the second state, optionally visually noting the change in the thermochromic material to ensure the application site is reduced in temperature by evaporation of the vapocoolant, if so employed. In another aspect, the thermochromic material is placed on the back side of the peel-away pull tab so that the user can apply, e.g., swipe, the thermochromic material in proximity to the skin application site. Alternatively, a temperature sensor as discussed above can be employed to approximate or verify the skin application site is brought to a desired temperature or below a temperature threshold to facilitate reduced pain in a subsequent invasive procedure.

In one variation, the container 120, 220 is not refillable. As such, when it is used at least once or depleted of contents, the dispenser 10, 20 is discardable. In another variation, valve member 132, 232 connected to the container 120, 220 may be used to refill the container 120, 220 with contents.

The fixed relationship between the stop member and the actuator in combination with the collapsible chamber configuration of the housing provides for the actuator, in a first state, to remain in a sealed relationship with the seal member of the container outlet and to allow transition to a second state where the first portion of the housing is urged towards the second portion of the housing (via linear translation of the first and the second portions of the housing along the longitudinal axis A of the housing) causing the actuator to engage the stop member and constraining it from forward movement with respect to the container. Thus, with the actuator 134, 234 received within the recess of the stop member, the actuator 134, 234 is also constrained from further longitudinal movement. Thus, when the user continues to translate the first and second portions towards each other, the container moves with respect to the actuator 134, 234 to open the seal member 136, 236 and cause dispensing of the vapocoolant from the container 120, 220 through the orifice/nozzle 135, 235 onto the application site.

When the user releases the first portion of the housing a spring or other energy storage means (not shown) can be employed to move the first portion and the container back to the first state. If desired, a cam device (not shown) may be associated with the first portion and/or stop member so that a predetermined metered dose of vapocoolant is dispensed each time the first portion of the housing is pushed or turned, regardless of how long it is held in the depressed position or turned. Such cam devices are generally known.

With particular reference to FIGS. 4 - 7, it is seen that wing-like appendages 205 (e.g., two) extend in opposite directions close to the proximal end of the housing portions 101, 201. These appendages include respective distally facing arcuate surfaces to receive fingers of the user on either side of first housing 201 to facilitate secure gripping of the dispenser 10, 20, whereupon the thumb may be used to depress the first housing portion 201 to collapse the collapsible chamber and dispense the contents of container 220.

In one manner of use dispenser 10, 20, is disposable, where the container 120, 220 is not replaceable due to the threaded relationship of the housing portions 101, 102 or coupled relationship of housing portions 201, 202. Thus, when the container 120, 220 is used or emptied, the entire dispenser 10, 20 can be discarded. Alternatively, in one manner of use of the dispenser 10, 20, is reusable, where container 120, 220 is refilled or replaced, either via the valve member 132, 232 or by disassembly and replacing of the container 120, 220 and reassembly.

Once positioned about the application site, the collapsible chamber 99 is collapsed by urging first housing portion 201 into second housing portion 202, to cause the actuator 134, 234 to engage stop member 112, 210 at recess 116, 216 and to release seal member 136, 236 from outlet of container 120, 220 to permit release of the contents for a period of time.

In one aspect, cover member 218 can remain connected to the housing portion 202 after it is released in a manner similar to that described above. Thus, as shown in Fig. 7A, connector feature 217 is connected to housing portion 202 and cover member 218. Connector feature 217 can be one or more of a tether, a living hinge, hinge pin, cord, or chain, and can be made of the same material as that of the housing portion. In one aspect, the prep pad is located on the outside of the connected lid and a peel away tab is positioned on the inside surface of the lid and may contain a bandage.

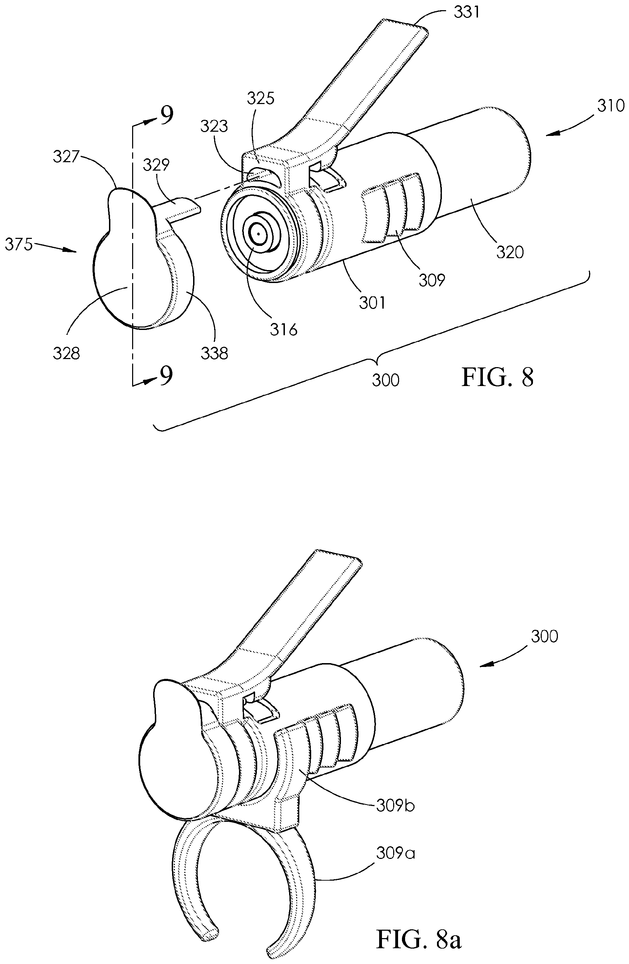

With reference now to FIGs. 8 - 10, dispenser 300 is now described in relation to longitudinal axis A. Dispenser 300 is configured for providing a one-handed, "pen-like" or "air-brush-like" feel to the user. As shown, actuator member 331, flexibly attached at an acute angle to stop body 340, has protruding member 337. Actuator member 331 can be flexibly attached with a living hinge or a conventional hinge. Actuator member 331 can include finger grips or other textured surfaces for ergonomic control during use so as to allow the user one-handed control. Housing 301 has a generally cylindrical interior to receive container 320 having contents for dispensing.

Dispenser 300 has cover assembly 375 and housing body assembly 310. Cover assembly 375 includes cover body 338 configured to receive prep pad 345, which can be an alcohol prep pad or other antiseptic. Pad 345 can be secured to cover body 338 surface and covered by peel away cover 328 with tab 327.

Actuator member 331 extends from the outer surface of stop body 340 positioned between a pair of finger guides 309 located about the outer surface of housing 301. Housing 301 has a partially closed end 325 having through-slot 323 through the stop body 340 configured to permit elongated tab 329 of cover body 338 to extend through stop body 340 and allow distal end 329a to engage surface 337a of protruding member 337 of actuator member 331. Distal end 329a has features securing it to stop body 340, such as a tab or cam lobe-like feature.

In another aspect, as shown in Fig. 8A, a ring-like feature projecting from the housing of dispenser 300. The ring-like feature is configured to accommodate a human finger. The ring-like feature can be comprised of two, semi-rigid, flexible curved projections 309a that have distal ends that are closely spaced that allow for a universal fit of a human finger of various sizes. Thus, the user can maintain the dispenser on a digit of one hand until needed for dispensing. The projections 309a can be configured with snap-on features 309b to reversibly snap-fit on the outer surface of the housing 320 for re-use.

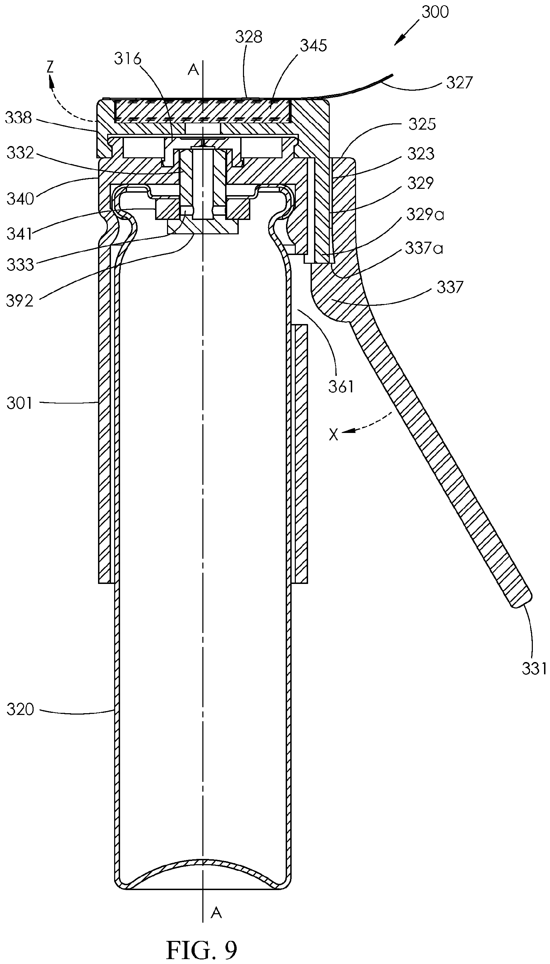

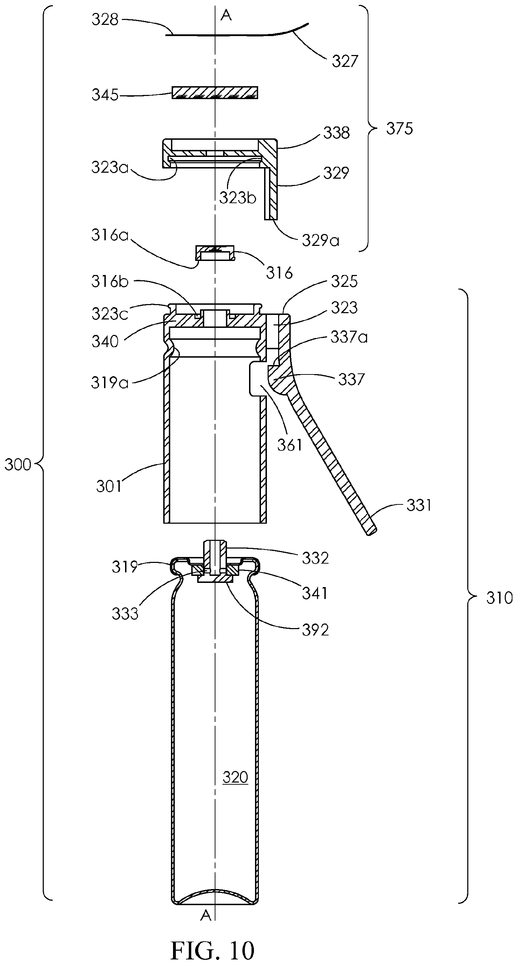

With reference to FIGs. 9 and 10, dispenser 300 is shown along section line 9-9 of Fig. 8. Protruding member 337 has surface 337a for engaging distal end 329a of elongated tab 329 from cover assembly 375. In a first state, actuator member 331 is moved by user towards housing 301 in a direction generally transverse to the axis A-A, surface 337a engages distal end 329a of elongated tab 329 causing cover assembly 375 to release from housing 301. In a second state, further translation of actuator member 331 towards housing 301 causes member 337 to enter opening 361 of housing 301 contacting container 320 to pivot or tilt the container in housing 301. As the container 320 is tilted actuating tilt-valve 392 causes valve stem 332, which is securely positioned in stop body 340, to separate, e.g., tilt, from sealing member 341 (flange) allowing vapocoolant from container 320 to dispense through opening 333 in valve stem 332 and nozzle 316. Nozzle 316 has proximal end 316a secured in stop body 340 in recess 316b. Cover body 338 is configured with recess features 323a, 323b sized to accommodate features 323c of housing 301. Container 320 can have annular grooved neck 319, which can be received by features present in the interior surface of housing 301, such as an annular protrusion 319a.

Tilt-valve assembly 392 can be fixedly coupled to container 320 using conventional techniques, such as a peripheral annular channel encasing a peripheral container bead defining a perimeter of the container top opening (not shown). Rigid valve stem 332 can be engaged snugly in a central bore of a grommet (not shown) held in place by for example annular flanges sandwiching the upper and lower portions of the grommet. The valve stem can be formed by a hollow tube closed at a first end by a base forming a flange of diameter greater than the one of the inner bore of the grommet and which upper surface of the base flange is suitable for sealing against the lower surface of the grommet flange. The lateral wall of the tubular portion of the stem generally comprise one or more openings 333 providing fluid communication between the inner bore of the stem 332 with the interface between the stem base and the grommet flange. By tilting and/or pressing sideways the portion of the valve stem extending out of the grommet via the actuator member 331 and member 337 moving or tilting container 320 sideways, the sealing interface between the grommet flange and the valve base is broken or disrupted thus bringing in fluid communication the inner bore of the valve stem with the vapocoolant contained in the container 320. Since container 320 is pressurized slightly, the vapocoolant of the container is dispensed through the valve and nozzle 316. When closed, the valve is configured such that no moisture or air enters the container. Other valve assemblies or designs can be used.

With the third embodiment having been described in detail above, a method of operation will now be explained. Container 320 has vapocoolant contained therein by tilt-valve 392 and is received by stop body 340 integral with housing 301 releasably coupled to cover assembly 375. In one aspect, peel away cover 328 is removed by pulling on tab 327 exposing alcohol pad 345 for sterilizing site on patient to be cooled prior to accessing. Alternatively, the entire cover assembly 375 can be removed without exposing alcohol pad 345. Actuator member 331 is translated towards container 320 as shown by arrow X, by allowing surface 337a to engage distal end 329a of elongated tab 329 and urging recess feature 323b to slide over feature 323c of stop body 340 so as to release cover assembly 375 from housing 301, as shown by arrow Z (FIG. 9). With cover assembly 375 separated from housing 301, actuator member 331 can be further translated (either continuously, e.g., one-stage, or sequentially, e.g., two-stage) towards container 320 causing member 337 to contact container 320 by entering through opening 361, which causes tilt-valve 392 to release vapocoolant through nozzle 316. User can controllable grasp dispenser 300 with a single hand, with the help of finger guides 309, such that the user can manipulate the dispenser 300 and a syringe or blade with the other hand in a controlled manner. Thus, the present dispenser avoids needing a "third hand" or the need to put one device down so as to access and use another device.

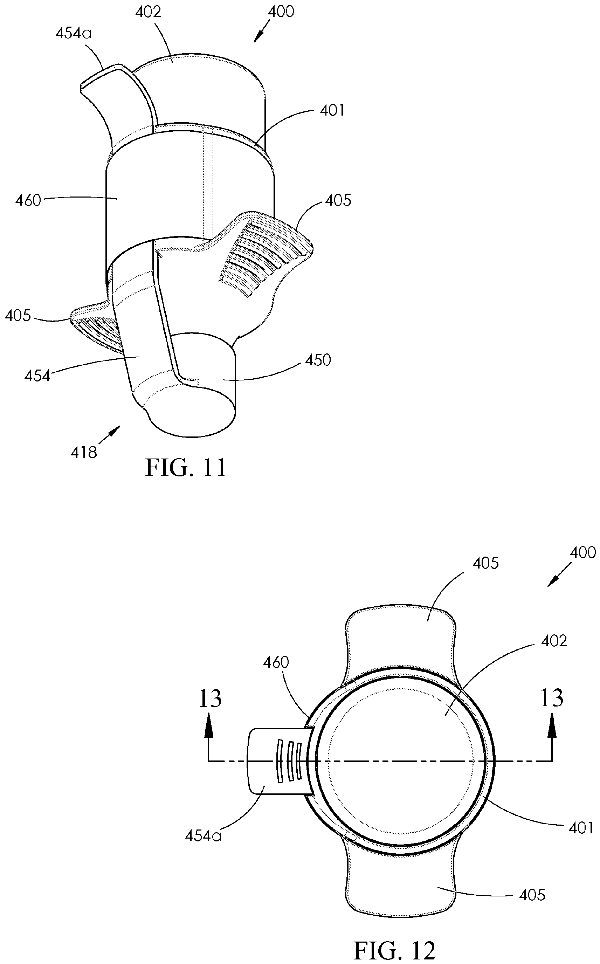

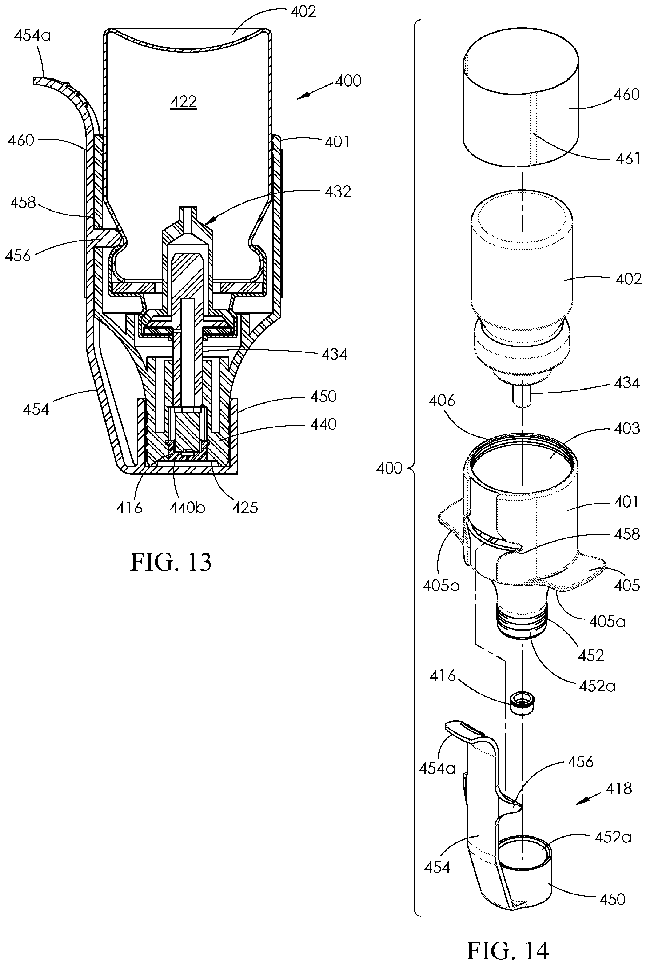

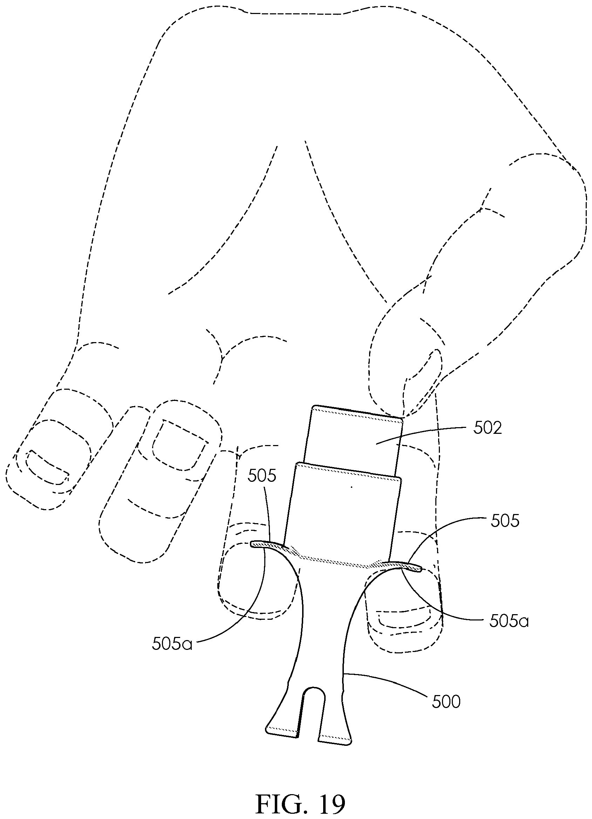

Referring to FIGs. 11 - 14 another embodiment of the dispenser is shown generally at 400. The dispenser has a housing 401 comprising a cavity 403 that retains the container 402 filled with content 422 under pressure. Housing 401 has a partially closed end 425. Nozzle 416 is secured in stop body 440 in recess 440b. Nozzle 416 may comprise a micromist member to split the flow of the vapocoolant so that it can recombine in one or more channels making a vortex that aids in making a finer mist of vapocoolant. The internal cavity 403 of the housing 401 may be provided with a peripheral seal 406 that engages the container 402 to retain the container in the housing and that isolates the interior of the housing from the ambient environment. A valve member 432 is provided in an opening in the container 402 that includes an actuator 434 that is engaged with a recess in the stop body 440 such that when a force is applied to the container 402 by a user that depresses the container the valve member 432 opens as previously described to dispense contents from the container 402 to the nozzle 416 where it is dispensed from the dispenser. In one embodiment the housing is provided with wing-like appendages 405 (e.g., two) that extend in opposite directions from housing 401. These appendages 405 include respective distally facing surfaces 405a to receive fingers of the user on either side of housing 401 to facilitate secure gripping of the dispenser whereupon the thumb may be used to depress the container to open the valve and dispense the contents of container as shown in FIG. 19.

In some embodiments, housing 401 comprises artwork or other indicia. In yet other embodiments, housing 401 comprises an integrated bandage or a sticker for use with pediatric patients.

A cover assembly 418 is provided that includes a cap 450 that fits over the end or neck 452 of housing 401 to close the nozzle 416 providing for aseptic or semi-aseptic assembly. The cap 450 and/or the neck 452 may comprise seal members 452a that engage the other one of the cap and neck to create a relatively tight fit between the cap and the neck to retain the cap on the housing. A handle 454 is attached to the cap 450 to provide easy removal of the cap 450. In one embodiment the cap 450 and handle 454 are made of plastic and the handle is configured in a tether-like manner such that the handle 454 is relatively flexible and may be manipulated away from the cap. The handle 454 comprises a protrusion 456 that engages a slot 458 formed in housing 401 such that the handle may be fixed in position relative to the housing by inserting the protrusion 456 into the slot 458. The slot and protrusion are dimensioned and configured such that the engagement of the protrusion with the slot fixes the handle in position relative to the housing but allows a user to remove the handle from the slot to allow the cap to be removed. In one embodiment the handle and slot are configured usch that the handle rests against the wall of the housing 401 during storage to minimize the footprint of the dispenser. To remove the cover assembly 418, the user grasps the handle 454 and pulls the protrusion 456 from the slot 458. The user may then apply a force to the handle to pull the cap 450 from the neck 452 of housing 401.

In order to maintain the handle in the storage position of FIGs. 10 and 11 when the dispenser is not in use, a wrap or seal 460 may be applied around the handle 454 and the housing 401 that prevents inadvertent removal of the cap. The wrap or seal 460 may comprise a plastic, foil or other similar band or sheet that wraps around the handle and housing. The wrap or seal 460 may comprise artwork or indicia and/or may be integrated with a bandage or a sticker. The wrap or seal may be easily broken by grasping the exposed distal end 454a of handle 454 and pulling the handle downward as viewed in the drawings such that the handle breaks the wrap or seal. Once the seal is broken the handle may be pulled to release the cap 450 from the neck of the housing. Suitable score lines or other frangible structure 461 may be provided on the seal to facilitate fracture of the seal when the handle is pulled.

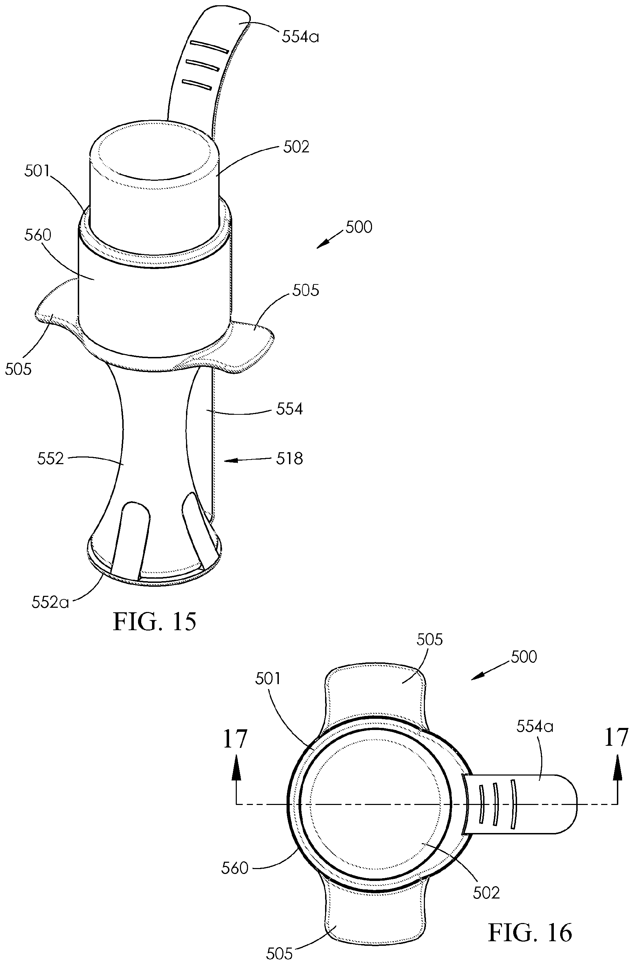

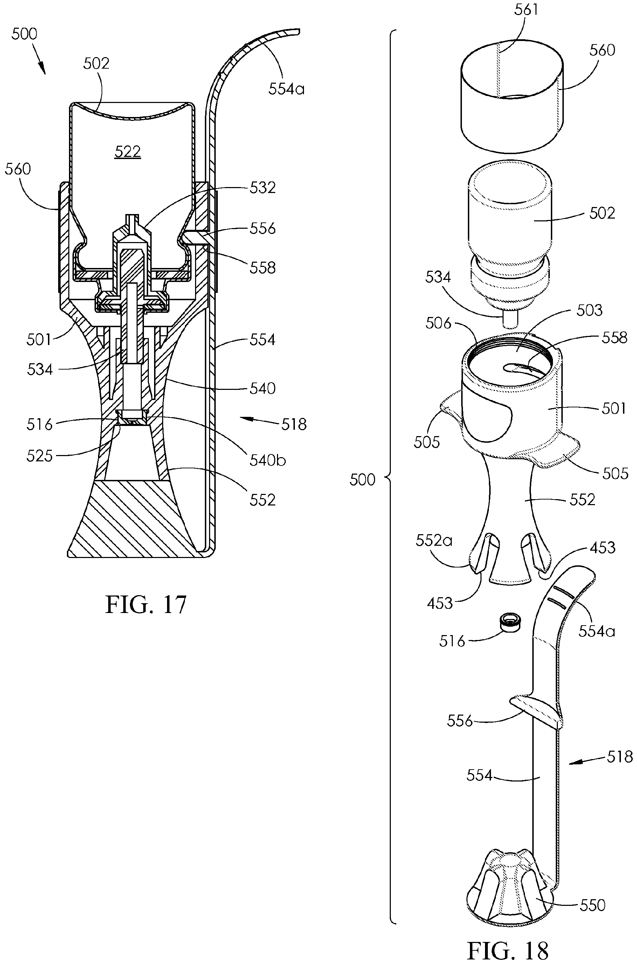

Referring to FIGs. 15 - 19 another embodiment of the dispenser is shown generally at 500 that is similar to the device described above with reference to FIGs. 11 - 14. The dispenser has a housing 501 comprising a cavity 503 that retains the container 502 filled with content 522 under pressure. Housing 501 has a partially closed end 525. Nozzle 516 is secured in stop body 540 in recess 540b. The internal cavity 503 of the housing 501 may be provided with a peripheral seal 506 that engages the container 502 to retain the container in the housing and that isolates the interior of the housing from the ambient environment. A valve member 532 is provided in an opening in the container 502 that includes an actuator 534 that is engaged with a recess in the stop body 540 such that when a force is applied to the container 502 by a user that depresses the container the valve member 532 opens as previously described to dispense contents from the container 502 to the nozzle 516 where it is dispensed from the dispenser. In one embodiment the housing is provided with wing-like appendages 505 (e.g., two) that extend in opposite directions from housing 501. These appendages 505 include respective distally facing surfaces 505a to receive fingers of the user on either side of housing 501 to facilitate secure gripping of the dispenser whereupon the thumb may be used to depress the container to open the valve and dispense the contents of container as shown in FIG. 19.

A cover assembly 518 is provided that includes a cap 550 that fits into the end or neck 452 of housing 501 to close the nozzle 516 providing for aseptic or semi-aseptic assembly. The cap 550 may fit into slots 453 formed in the neck 452 to create a relatively tight fit between the cap and the neck to retain the cap on the housing. A handle 554 is attached to the cap 550 to provide easy removal of the cap 550. In one embodiment the cap 550 and handle 554 are made of plastic and the handle is configured in a tether-like manner such that the handle 554 is relatively flexible and may be manipulated away from the cap. The handle 554 comprises a protrusion 556 that engages a slot 558 formed in housing 501 such that the handle may be fixed in position relative to the housing by inserting the protrusion 556 into the slot 558. The slot and protrusion are dimensioned and configured such that the engagement of the protrusion with the slot fixes the handle in position relative to the housing but allows a user to remove the handle from the slot to allow the cap to be removed. In one embodiment the handle and slot are configured such that the handle rests against the wall of the housing 501 during storage to minimize the foot print of the dispenser. To remove the cover assembly 518, the user grasps the handle 554 and pulls the protrusion 556 from the slot 558. The user may then apply a force to the handle to pull the cap 550 from the neck 552 of housing 501.

In order to maintain the handle in the storage position of FIGs. 15 and 16 when the dispenser is not in use, a wrap or seal 560 may be applied around the handle 554 and the housing 501 that prevents inadvertent removal of the cap. The wrap or seal 560 may comprise a plastic, foil or other similar band or sheet that wraps around the handle and housing. The wrap or seal may be easily broken by grasping the exposed distal end 554a of handle 554 and pulling the handle downward as viewed in the drawings such that the handle breaks the wrap or seal. Once the seal is broken the handle may be pulled to release the cap 550 from the neck of the housing. Suitable score lines or other frangible structure 561 may be provided on the seal to facilitate fracture of the seal when the handle is pulled.

In the embodiment of FIGs. 15 - 19 the neck 552 of the housing 501 is elongated such that the distal end 552a of neck 552 is disposed a distance from the nozzle 516. The distance between the end 552a of the neck and the nozzle may be adjusted by making the neck relatively longer or shorter such that the distance between the nozzle and the application site may be changed. By varying this distance the temperature of the content applied to the site may be varied or controlled. For example, a longer neck will allow the content to warm up more before contacting the application site as compared to a shorter neck. In this manner the temperature of the content at the application site may be controlled for different vapocoolants, different applications and/or uses or the like.

When the present dispenser is employed to dispense vapocoolant, the location on the skin of the patient where topical anesthesia is to take place is determined and the dispenser 10, 20, or 300 is positioned at a desired location above the skin or in contact with the skin. In one aspect, the dispenser is actuated approximately 4 to 9 cm (1.5 to 4 inches) away from the application site. In certain aspects, the dispenser can be placed directly in contact with the application site prior to actuation of the valve member to release the vapocoolant. In other aspects, the dispenser can be placed above the application site and maintained in a stationary position, or alternatively, be swept in a back-and-forth linear fashion or in a circular motion above the application site.

In one aspect, the dispenser is configured to be manipulated by one hand of the user such that the other hand is free to proceed to inject or otherwise act upon the application site. In this aspect, it is possible to facilitate a repetitious but ergonomic "spray-inject" sequence, for example, for the treatment of varicose veins, where multiple injections are necessary along an extended length of a leg. In this regard, it is not necessary for constant manipulation of the dispenser and medical device by the health practitioner, thus facilitating a more efficient and safe procedural end result.

In one aspect, the dispenser can be configured to release vapocoolant for a time of approximately 1 to 10 seconds, or longer. In one aspect the dispenser is configured to release vapocoolant for 1-2 seconds, 3 to 5 seconds, 6-10 seconds, or longer. In other aspects, the dispenser is configured to release vapocoolant continuously. In other aspects, the dispenser is configured to release vapocoolant intermittently, for example, metered amounts of vapocoolant dispensed at predetermined durations of time using a cam device.

Either while the vapocoolant is flowing or just after the flow of the vapocoolant is stopped, the skin can then be accessed e.g., penetrated by a needle of the syringe to a desired depth, insertion of catheter or other medical device or the like. In using the dispenser disclosed, various types of dermal/sub-dermal accesses may be subsequently employed, such as subcutaneous, intramuscular or intradermal.

If desired, additional injections may be carried out at the same or at different locations. During the process of injection multiple injections at the same or different sites, additional vapocoolant may be dispensed as desired to provide or maintain the numbness of the skin at that location. If during the process of injection, the container runs out of vapocoolant, it may be removed from the housing and replaced with a replacement container or be refilled. When the injection process is completed, the dispenser can be properly discarded or the dispenser may be refilled and/or re-used with other procedures.

The devices and assemblies presently described provide for a configuration and function that is advantageous for a person in need of numbing an injection site prior to self-injection, for example injections in proximity to the buttocks or other locations such as the lower legs, back, shoulders, etc.

As such, in this way, the present disclosure provides an effective way of dispensing a vapocoolant onto the skin of a patient, e.g., that would benefit from the anesthetic effect of a vapocoolant and/or where a medical treatment is to take place that would benefit from the anesthetic effect of a vapocoolant, so that an injection or other invasive procedure is painless or less painful. The present disclosure is not limited for use in association with injections for aesthetic enhancement. Rather, it may be used in combination with any invasive procedure and/or with other medical devices, or for use with an injector, catheter, inserter, or other medical device that is invasive and likely to result in pain to the patient.

As such, it is intended that the present disclosure only be limited by the terms of the appended claims.

## Claims

1. A vapocoolant dispenser, comprising:
a housing (200) having a first portion (201) and a second portion (202) coupled with the first portion defining a collapsible chamber (99);
a container (220) configured to contain contents under pressure, the container positioned in the housing (200);
a valve member (232) for selectively opening the container, the valve member comprising:
an actuator portion (234) configured to extend from the container; and
a seal member (236) sealably engaged with the container; and
a stop member (210) positioned in the second portion (202) of the housing (200) and configured to engage the actuator portion (234);
such that when the collapsible chamber is collapsed, the actuator portion is engaged by the stop member, disengaging the seal member from the container for releasing the contents,
wherein one of the first portion and the second portion has an inner perimeter of larger dimension than an outer perimeter of the other one of the first portion and the second portion, and wherein the first portion is configured to translate along a common longitudinal axis with that of the second portion so as to collapse the collapsible chamber, wherein the container is fixedly positioned in the second portion of the housing;
a cover assembly (218) is releasably attached to the second portion (202)
**characterized in that**
the cover assembly comprises a prep pad (250) having antiseptic;
wherein the cover assembly comprises a rim (221) configured to engage with tab members (214) of the first portion (201) and the internal surface of the cover assembly (218) and the external surface of the second portion (202) are formed with mating retaining members (215); and
a rigid skirt (207) integral within and extending about an annular edge of the second portion;
wherein the cover assembly is released with the following mechanism: when the housing portion (201) is depressed to begin the collapse of chamber (99), the tab members (214) engage the rim (221) and cause the retaining members (215) to release such that the cover assembly (218) is released from the second housing portion (202).

2. The dispenser of claim 1, further comprising an absorbent material having an inward facing side and an outward skin-facing side, the absorbent material positioned distally from the valve member to receive the contents on the inward facing side.

3. The dispenser of claim 1, wherein the stop member comprises a surface having a recess (216) dimensioned to receive at least a portion of an outlet (230) and at least a portion of the valve member (232).

4. The dispenser of claim 1, wherein the rigid skirt terminates in an annular ring (209), and where the dispenser further comprising an absorbent material fixedly held to the annular ring, the absorbent material having an inward facing side and an outward skin-facing side, the absorbent material positioned distally from the valve member and configured to receive the contents on the inward facing side.

5. The dispenser of claim 1, wherein the cover assembly is configured to remain connected to the housing upon release.

6. The dispenser of any one of claims 1-5, wherein the dispenser is configured to transition to:
a first state wherein the contents are contained within the container and the cover assembly is released from the second portion (202) of the housing (200); and
a second state wherein the first and the second portions are urged in proximity with each other so as to collapse the collapsible chamber (99) and engage the actuator ( 234) with the stop member (210) disengaging the seal member (236) from an outlet (230) and allowing an amount of the contents to be released.

7. The dispenser of claim 6, wherein the cover assembly is configured to disengage from the housing prior to release of the contents.

8. The dispenser of claim 7, wherein the contents comprise a vapocoolant and a metered amount of the vapocoolant is released or wherein an amount of vapocoolant is continuously released.

## Patentansprüche

1. Kältesprayspender, umfassend:
ein Gehäuse (200) mit einem ersten Abschnitt (201) und einem zweiten Abschnitt (202), der mit dem ersten Abschnitt verkuppelt ist, wodurch eine klappbare Kammer (99) definiert ist;
einen Behälter (220), der zum Enthalten von Inhalten unter Druck konfiguriert ist, wobei der Behälter im Gehäuse (200) angeordnet ist;
ein Ventilelement (232) zum selektiven Öffnen des Behälters, wobei das Ventilelement umfasst:
einen Betätigungsabschnitt (234), der dazu konfiguriert ist, sich aus dem Behälter zu erstrecken; und
ein Dichtungselement (236), das abdichtend mit dem Behälter in Eingriff steht; und
ein Anschlagelement (210), das im zweiten Abschnitt (202) des Gehäuses (200) angeordnet ist und dazu konfiguriert ist, den Betätigungsabschnitt (234) in Eingriff zu nehmen;
sodass, wenn die klappbare Kammer zusammengeklappt ist, der Betätigungsabschnitt durch das Anschlagelement in Eingriff genommen ist, wodurch das Dichtungselement zum Freigeben der Inhalte gelöst wird,
wobei einer des ersten Abschnitts und des zweiten Abschnitts einen Innendurchmesser mit größerer Abmessung als ein Außendurchmesser des anderen des ersten Abschnitts und des zweiten Abschnitts aufweist, und wobei der erste Abschnitt dazu konfiguriert ist, sich entlang einer gemeinsamen Längsachse mit jener des zweiten Abschnitts zu verschieben, um die klappbare Kammer zusammenzuklappen, wobei der Behälter starr im zweiten Abschnitt des Gehäuses angeordnet ist;
wobei eine Abdeckbaugruppe (218) lösbar am zweiten Abschnitt (202) angebracht ist,
**dadurch gekennzeichnet, dass**
die Abdeckbaugruppe ein Prep-Pad (250) mit Antiseptikum umfasst;
wobei die Abdeckbaugruppe einen Rand (221) umfasst, der dazu konfiguriert ist, mit Zungengliedern (214) des ersten Abschnitts (201) in Eingriff zu treten, und die Innenfläche der Abdeckbaugruppe (218) und die Außenfläche des zweiten Abschnitts (202) mit zusammenpassenden Haltegliedern (215) ausgebildet sind; und
eine starre Schürze (207), die innerhalb einer ringförmigen Kante des zweiten Abschnitts einstückig ist und darum herum verläuft;
wobei die Abdeckbaugruppe mit dem folgenden Mechanismus gelöst wird: wenn der Gehäuseabschnitt (201) niedergedrückt wird, um das Zusammenklappen der Kammer (99) zu beginnen, nehmen die Zungenglieder (214) den Rand (221) in Eingriff und bewirken, dass sich die Halteglieder (215) lösen, sodass die Abdeckbaugruppe (218) vom zweiten Gehäuseabschnitt (202) gelöst wird.

2. Spender nach Anspruch 1, ferner umfassend ein Absorptionsmaterial mit einer nach innen gerichteten Seite und einer nach außen, zur Haut hin gerichteten Seite, wobei das Absorptionsmaterial distal zum Ventilelement angeordnet ist, um die Inhalte auf der nach innen gerichteten Seite aufzunehmen.

3. Spender nach Anspruch 1, wobei das Anschlagelement eine Oberfläche mit einer Aussparung (216) umfasst, die zum Aufnehmen von zumindest einem Abschnitt eines Auslasses (230) und zumindest einem Abschnitt des Ventilelements (232) bemessen ist.

4. Spender nach Anspruch 1, wobei die starre Schürze in einem Kreisring (209) endet, und wobei der Spender ferner ein Absorptionsmaterial umfasst, das starr am Kreisring gehalten ist, wobei das Absorptionsmaterial eine nach innen gerichtete Seite und eine nach außen, zur Haut hin gerichtete Seite aufweist, wobei das Absorptionsmaterial distal zum Ventilelement angeordnet ist und dazu konfiguriert ist, die Inhalte auf der nach innen gerichteten Seite aufzunehmen.

5. Spender nach Anspruch 1, wobei die Abdeckbaugruppe dazu konfiguriert ist, nach dem Lösen mit dem Gehäuse verbunden zu bleiben.

6. Spender nach einem der Ansprüche 1 bis 5, wobei der Spender dazu konfiguriert ist, zu Folgendem überzugehen:
in einen ersten Zustand, in dem die Inhalte im Behälter enthalten sind und die Abdeckbaugruppe vom zweiten Abschnitt (202) des Gehäuses (200) gelöst wird; und
in einen zweiten Zustand, in dem der erste und zweite Abschnitt nahe aneinander gedrängt werden, um die klappbare Kammer (99) zusammenzuklappen und das Betätigungselement (234) in Eingriff zu nehmen, wobei das Anschlagelement (210) das Dichtungselement (236) aus einem Auslass (230) löst und ermöglicht, dass eine Menge der Inhalte freigegeben wird.

7. Spender nach Anspruch 6, wobei die Abdeckbaugruppe dazu konfiguriert ist, sich vor der Freigabe der Inhalte vom Gehäuse zu lösen.

8. Spender nach Anspruch 7, wobei die Inhalte ein Kältespray umfassen und eine dosierte Menge des Kältesprays freigegeben wird, oder wobei eine Kältespraymenge fortlaufend freigegeben wird.

## Revendications

1. Distributeur de vaporéfrigérant, comprenant :
un boîtier (200) ayant une première partie (201) et une seconde partie (202) couplée à la première partie définissant une chambre pliable (99) ;
un récipient (220) configuré pour contenir des contenus sous pression, le récipient étant positionné dans le boîtier (200) ;
un élément de valve (232) pour sélectivement ouvrir le récipient, l'élément de valve comprenant :
une partie actionneur (234) configurée pour s'étendre à partir du récipient ; et
un élément de joint (236) en prise hermétique avec le récipient ; et
un élément d'arrêt (210) positionné dans la seconde partie (202) du boîtier (200) et configuré pour se mettre en prise avec la partie actionneur (234) ;
de telle sorte que lorsque la chambre pliable est pliée, la partie actionneur est mise en prise par l'élément d'arrêt, désengageant l'élément de joint du récipient pour libérer les contenus,
dans lequel l'une de la première partie et de la seconde partie a un périmètre intérieur d'une dimension supérieure à celle d'un périmètre extérieur de l'autre de la première partie et de la seconde partie, et dans lequel la première partie est configurée pour se translater le long d'un axe longitudinal commun avec celui de la seconde partie pour plier la chambre pliable, dans lequel le récipient est positionné de manière fixe dans la seconde partie du boîtier ;
un ensemble de couvercle (218) est fixé de manière amovible à la seconde partie (202),
**caractérisé en ce que**
l'ensemble de couvercle comprend un tampon prep (250) ayant un antiseptique ;
dans lequel l'ensemble de couvercle comprend un bord (221) configuré pour se mettre en prise avec des éléments de languettes (214) de la première partie (201) et la surface interne de l'ensemble de couvercle (218) et la surface externe de la seconde partie (202) sont formées avec des éléments de retenue (215) s'appariant ; et
une jupe rigide (207) formant partie intégrante à l'intérieur et s'étendant autour d'un bord annulaire de la seconde partie ;
dans lequel l'ensemble de couvercle est libéré avec le mécanisme suivant : lorsque la partie de boîtier (201) est enfoncée pour commencer le pliage de la chambre (99), les éléments de languettes (214) se mettent en prise avec le bord (221) et amènent les éléments de retenue (215) à se libérer de telle sorte que l'ensemble de couvercle (218) est libéré de la seconde partie de boîtier (202).

2. Distributeur selon la revendication 1, comprenant en outre un matériau absorbant ayant un côté face à l'intérieur et un côté extérieur face à la peau, le matériau absorbant étant positionné à distance de l'élément de valve pour recevoir les contenus sur le côté face à l'intérieur.

3. Distributeur selon la revendication 1, dans lequel l'élément d'arrêt comprend une surface ayant une cavité (216) dimensionnée pour recevoir au moins une partie d'un orifice de sortie (230) et au moins une partie de l'élément de valve (232).

4. Distributeur selon la revendication 1, dans lequel la jupe rigide se termine en une bague annulaire (209), et dans lequel le distributeur comprend en outre un matériau absorbant maintenu de manière fixe sur la bague annulaire, le matériau absorbant ayant un côté face à l'intérieur et un côté extérieur face à la peau, le matériau absorbant étant positionné à distance de l'élément de valve et configuré pour recevoir les contenus sur le côté face à l'intérieur.

5. Distributeur selon la revendication 1, dans lequel l'ensemble de couvercle est configuré pour rester raccordé au boîtier lors de la libération.

6. Distributeur selon l'une quelconque des revendications 1 à 5, dans lequel le distributeur est configuré pour passer :
d'un premier état dans lequel les contenus sont contenus à l'intérieur du récipient et l'ensemble de couvercle est libéré de la seconde partie (202) du boîtier (200) ; et
un second état dans lequel les première et seconde parties sont poussées à proximité l'une de l'autre de manière à plier la chambre pliable (99) et à mettre en prise l'actionneur (234) avec l'élément d'arrêt (210) désengageant l'élément de joint (236) d'un orifice de sortie (230) et permettant la libération d'une quantité des contenus.

7. Distributeur selon la revendication 6, dans lequel l'ensemble de couvercle est configuré pour se désengager du boîtier avant de libérer les contenus.

8. Distributeur selon la revendication 7, dans lequel les contenus contiennent un vaporéfrigérant et une quantité dosée du vaporéfrigérant est libérée ou dans lequel une quantité du vaporéfrigérant est libérée en continu.
